Europäisches Patentamt

**European Patent Office**

Office européen des brevets

(11) Publication number: **0 028 133**
A2

(12) **EUROPEAN PATENT APPLICATION**

(21) Application number: **80303757.1**

(51) Int. Cl.³: **G 01 N 33/54**

(22) Date of filing: **23.10.80**

(30) Priority: **26.10.79 US 88696**

(43) Date of publication of application: **06.05.81**
Bulletin 81/18

(84) Designated Contracting States: **CH DE FR GB IT LI**

(71) Applicant: **DYNASCIENCES CORPORATION,**
**10880 Wilshire Boulevard, Los Angeles**
**California 90024 (US)**

(72) Inventor: **Cooper, Harold R., 8383 Inspiration Avenue,**
**Walkersville Maryland 21793 (US)**
Inventor: **O'Beirne, Andrew, 9403 Farmingdale Avenue,**
**Walkersville Maryland 21793 (US)**

(74) Representative: **Wilson, Joseph Martin, WITHERS &**
**ROGERS 4 Dyer's Buildings Holborn, London EC1N 2JT**
**(GB)**

(54) **Method of detecting and quantitating haptens and antigens.**

㉗ The method comprises contacting a sample containing an unknown quantity of a first immuno-reactive substance selected from haptens, antigens and mixtures thereof, to be detected and quantitated, with (a) a known quantity of a reagent containing a known concentration of an antibody specific for that first immuno-reactive substance, and (b) a solid phase having a known concentration of a second immuno-reactive substance selected from haptens, antigens and mixtures thereof reactible with the antibody to be passively adsorbed to the solid phase. The contacting is effected for a time sufficient to cause the first and second immuno-reactive substances to combine competitively with the antibody. The solid phase is then isolated, bearing with it the second immuno-reactive substance and the antibody bound thereto. That antibody is then labeled by any suitable means and thereafter the concentration of the labeling bound to the antibody is determined as a measure of the concentration of the antibody bound to the solid phase and as a measure of the first immuno-reactive substance initially present in the sample. The sample and the reagent containing the antibody are in the liquid phase and the sample and reagent may either be mixed together and reacted before contacting the solid phase or, alternately, the sample, reagent and solid phase may contact each other simultaneously for the first time. An enzyme or a radioactive material or the like may be used as the labeling agent. The method has improved sensitivity and adaptability and does not require the use of dangerous or expensive reagents.

- 1 -

# METHOD OF DETECTING AND QUANTITATING HAPTENS

# AND ANTIGENS

## Background of the Invention

### Field of the Invention

The present invention generally relates to test methods and more particularly to a method for detecting and quantitating haptens and antigens.

Haptens are classically measured by the competitive inhibition assay first described by Yalow and Berson (J. Clin. Invest. 39: 1157, 1960). This assay is a radioimmunoassay carried out by mixing a limiting amount of hapten-specific antibody with specified volumes of a sample containing an unknown amount of a hapten and a solution containing a known amount of the same hapten or an analog of the same hapten. Unlabeled and labeled haptens then compete for a limited number of antibody-binding sites. By separating the free and the antibody-bound labeled hapten into distinct fractions or phases and then measuring the amount of radioactivity in each of these two phases and then measuring the amount of radioactivity in each of these two phases, one can quantitatively determine the amount of hapten in the sample being tested. Phase separation of the free hapten and the antibody-bound hapten can be accomplished by several methods which are currently in practice. including the use of species-specific antiglobulin to precipitate hapten-antibody complexes. Charcoal, ion exchange resins and various other types of solid phases have also been used to bind specific antibody.

The general methodologies and principles which are utilized in the radioisotope immunossay methods employing phase separation to quantititate haptens have more recently also been applied to systems which employ reagents which are labeled with enzymes instead of radioisotopes. One such system is known as the enzyme-linked immunosorbent assay ( ELISA), utilizing enzyme-hapten conjugates and a plastic solid phase to which hapten-specific antibody is adsorbed to effect phase separation. As in the case of radioimmunoassays, sample hapten and enzyme-labeled hapten compete for a limited number of antibody-combining sites on the solid phase. The amount of antibody-bound labeled hapten (which has an inverse relationship to the amount of hapten in the unknown sample) is determined by measuring the enzymatic activity of the solid phase.

A second system known as the enzyme multiplication immunoassay technique (EMIT) is similar to ELISA and certain radioimmunoassays in that enzyme-labeled hapten and sample hapten compete for a limited number of antibody-binding sites. However, EMIT does not require phase separation because the enzyme-hapten conjugate is prepared in such a manner that the enzyme will not react with substrate when antibody is bound to the enzyme-hapten conjugate. Consequently, EMIT specifically measures the amount of free or unbound enzyme-hapten conjugate.

There are few examples of hapten assays in which hapten itself is bound to a solid phase. One such example is the competitive enzyme-linked immunoassay (CELIA) described by Yorde et al. (Clin. Chem. 22:1372, 1976). In the CELIA system free hapten in an unknown sample and hapten covalently bound to a solid phase, such as Sepharose beads which are cross-linked dextran, compete for a limiting number of antibody (1st antibody) binding sites in solution. The quantity of hapten in the unknown sample is determined by measuring the amount of specific antibody bound to the solid-phase. This measurement is accomplished by an enzymatic technique in which anti-globulin bound to 1st anti-body and anti-enzyme-enzyme immune complex are added in sequence. The anti-gloublin functions to bridge the bound hapten-specific antibody with the anti-enzyme-enzyme immune complex which is the indicator system. One then measures the enzymatic activity of the solid phase in the presence of substrate.

A radioimmunoassay, similar to CELIA in principle, has been developed by Zollinger and Mandrell (Infect. and Immun. 18:424, 1977) to serotype bacteria. In this procedure, specific typing antibody is preincubated with a heterologous antigen (distinct from hapten) preparation. The reaction mixture is then added to microtiter wells passively coated with antigen homologous to the typing antisera. The amount of antibody bound to the solid phase (which has an inverse relationship with the degree of antigenetic similarity shared by the heterologous test antigen and the solid phase homologous antigen) is determined by adding a radio-labeled anti-globulin and then measuring the amount of label bound to the solid phase.

The primary advantage of using a solid-phase hapten, that is a hapten bound to a solid phase, as in the CELIA procedure, is that the sensitivity of the hapten assay can be increased dramatically by using labeled anti-globulin as the indicator system rather than labeled hapten.

The reason for enhanced sensitivity pertains to the increased number of enzyme labels that can be linked to antibody relative to hapten molecules. In the former case, three to four enzyme molecules can be successfully linked to a single antibody resulting in a conjugate that will demonstrate both antibody and enzymatic activity. In contrast, a single hapten molecule can be labeled with only one enzyme molecule. In reality, the final product would be a population of enzyme molecules to which three or four hapten molecules are covalently bound.

If a solid phase hapten is employed in a competitive immunoassay, each hapten specific antibody reacting with the solid phase would result in either three to four or six to eight enzyme labels being bound depending on whether labeled hapten specific or labeled second antibody was employed. In contrast, if one employs

-4-

a hapten specific antibody solid phase in a competitive immuno-assay, each solid phase antibody could react with two labeled hapten molecules resulting in only two enzyme labels being bound. Thus, the enhanced sensitivity of the immunoassays which employ solid phase hapten and labeled antibody is due to increased amplification of the serologic reaction by virtue of having more labels involved on a unit basis. Unfortunately, simple inexpensive methods for attaching haptens to solid phases have not heretofore existed. However, the method set forth in copending U.S. patent application, Serial No. , filed , entitled "Method of Passively Adsorbing Immuno-Reactive Haptens to Solid Phases", of which Harold R. Cooper and Andrew O'Beirne are the inventors (said application being assigned to the assignee of the present application) for the first time provides a hapten-bearing carrier in a simple improved procedure.

Most of the existing immunoassay methods either require the use of radioisotope labels, which labels are potentially hazardous to the user, have short shelf lives and are expensive to measure due to the cost of the necessary instrumentation, or employ enzymes as labels. However, it is technically difficult to covalently bind the proper number of haptens to an enzyme and still retain adequate enzymatic activity. Certain of the immunoassays are relatively ineffective and certain of the assays are cumbersome, time-consuming and inaccurate. It therefore would be desirable to be able to provide an improved immunoassay method which would not require the use of a radioisotope- or enzyme-tagged hapten and would demonstrate increased sensitivity over methods employing such tagged haptens. The improved method should be adaptable to any given hapten assay.

## Summary of the Invention

The method of the present invention satisfies all the foregoing needs. The method is substantially as set forth in the Abstract above. It is simple, inexpensive, rapid, reproducible and effective. Moreover, it is safe, does not employ hazardous materials, and is two to twelve times as sensitive as assays employing tagged haptens. Moreover, it is readily adaptable to any given hapten assay.

The method involves contacting a sample of a first immuno-reactive substance selected from haptens, antigens and mixtures thereof, with two substances either simultaneously or sequentially, the substances being, first, a reagent containing a known concentration of an antibody specific for that first immuno-reactive substance and either simultanously or thereafter contacting the reagent and sample with a solid phase containing a known concentration of a second immuno-reactive substance selected from haptens, antigens and mixtures thereof, reactible with the antibody, that second immuno-reactive substance being passively adsorbed to the solid phase. The hapten-bearing said phase can be provided, for example, in accordance with the previously described co-pending U.S. patent application, Serial No.

The contacting is continued for a time sufficient to cause the first and second immuno-reactive substances to competitively combine with the antibody. The solid phase is then separated with the second immuno-ractive substance and combined antibody attached thereto. The antibody is then labeled by any suitable means and the extent of labeling is determined to detect the concentration of antibody and therefore the concentration of the first immuno-reactive substance in the sample. The labeling

-6-

can be done enzymatically although, if desired, radioactive labeling or other means can be employed. The method of the present invention is simple, rapid and inexpensive and it yields excellent results. Moreover, it is substantially more sensitive than conventional methods. Further features of the present invention are set forth in the following detailed description.

## Detailed Description

In accordance with the method of the present invention, an antigen or hapten is detected and quantitated. The method includes contacting a sample containing an unknown quantity of a first immuno-reactive substance selected from haptens, antigens and mixtures thereof with (a) a known quantity of a reagent containing a known concentration of an antibody specific for the first immuno-reactive substance and (b) a solid phase having a known concentration of a second immuno-reactive substance selected from haptens, antigens and mixtures thereof, reactible with the antibody, the second immuno-reactive substance being passively adsorbed to the solid phase. The contacting is for a time sufficient to cause the first and second immuno-reactive substances to combine competitively with the antibody.

The reagent referred to above which contains a known concentration of the antibody specific for the first immuno-reactive substance can comprise an aqueous solution, suspension or dispersion of the antibody. The antibody may be, for example, sheep anti-digoxin or rabbit anti-thyroxine. Normally, when carrying out the method of the present invention, such reagent contains between about 0.1 and about 0.001% by volume concentration of the antibody.

The solid phase referred to above can be prepared in any suitable manner, but preferably is prepared in accordance with the method specified in the aforesaid copending U.S. patent application, serial No.   . As set forth in detail in that application, the solid phase containing the second immuno-reactive substance selected from haptens, antigens and mixtures thereof is prepared by first covalently binding it to an appropriate macromolecular carrier. The hapten or antigen may be any one or more of a wide variety of materials, for example, drugs, hormones, antibiotics, pesticides and the like. The macromolecular carrier may be any suitable material which permits the hapten to be conveniently covalently bound to it, as by a selected coupling reagent. Typical macromolecular carriers may be, for example, bovine serum albumen, human serum albumen, egg albumen and polylysine. The coupling reagent normally used to effect the covalent binding between the hapten or antigen and the macromolecular carrier include glutaraldehyde, carbodiimides, diisocyanates, (O-carboxymethy-) hydroxylamines, anhydrides, diazonium compounds and dihalogenated dinitrobenzenes. Additional coupling reagents are as follows: hydrazides, azides, cyanogen bromide, N,N'-o-phenylenedimaleimide, m-maleimidobenzoyl-N hydroxysuccinimide ester and the like. Selection of the particular coupling reagent will depend upon the particular hapten or antigen and macromolecular carrier. Typical examples of such selections are set forth in the table below:

TABLE

| SAMPLE NO. | HAPTEN OR ANTIGEN | MACROMOLECULAR CARRIER | COUPLING REAGENT |
|---|---|---|---|
| 1 | gentamicin | bovine serum albumin | glutaraldehyde |
| 2 | digoxin | human serum albumin | periodate & sodium borohydride |
| 3 | thyroxine | bovine serum albumin | glutaraldehyde |
| 4 | triiodothyronine | bovine serum albumin | glutaraldehyde |

-8-

In effecting the covalent binding, the coupling reagent may be added to a solution or mixture of the hapten and/or antigen and macromolecular carrier, the concentration of the coupling reagent varying with the particular system involved.

As a typical example, in the sample listed as No. 1 in the table above, the coupling reagent is initially mixed with the macromolecular carrier to provide a concentration of coupling reagent to carrier of about 2:1 by weight, whereupon after a 3-hour incubation period at room temperature, the solution is chromatrographed on a column of cross-linked dextran (with a molecular weight exclusive limit of 2500) and the first 20 ml after the void volume collected. To the resulting mixture is added 200 mg of hapten which is incubated for about 20 hours at room temperature with stirring, whereupon 500 mg of glycine is added. The resulting mixture is incubated an additional two hours at room temperature, after which this conjugate solution is dialyzed for about three days against distilled water at room temperature and then lyophilized.

The carrier-hapten (antigen) conjugate prepared in accordance with the above is diluted in a buffer of carbonate-bicarbonate, pH 9.6 to a selected concentration of about 100 ng/ml. Other concentrations, pH's and buffers can be used. It is preferred that the conjugate in the liquid pahse be in a concentration of about 10 ng/ml to about 200 ng/ml when contacting the solid phase. Such solid phase may be any suitable material capable of adsorbing the conjugate within a reasonable amount of time. Preferably the material is one from which a standard test tube or microtiter well can be conveniently fabricated, for example, polystyrene, polyethylene or polyvinyl acetate. Such substrate or solid phase should be capable of adsorbing the conjugate within, for example, about 15 -- 24 hours.

-9-

After such adsorption, the solid phase is washed free of unbound conjugate, for example, by washing the microtiter plates containing microtiter wells with phosphate-buffered saline solution fortified with a surfactant or the like. Alternatively, distilled water can be used to wash the microtiter free of the unbound conjugate. Air drying or the like is then used to provide the solid phase in the finished storable form.

In carrying out the method of the present invention, the sample containing the first immuno-reactive substance to be detected and quantitated is contacted with the above-described reagent containing the antibody specific for that immuno-reactive substance, either simultaneously with the initial contact of the sample with the solid phase or before initial contact with the solid phase is effected. If the contact with the reagent precedes the contact with the solid phase, then the present method is said to be carried out in an indirect competitive manner. If the sample contacts the reagent and the solid phase for the first time and simultaneously, then a direct competitive technique is employed. The indirect competitive technique can be carried out merely by intimately mixing the reagent and sample together and allowing sufficient time, for example, about 0.5 to two hours, for the reaction to take place between the antibody and first immuno-reactive substance. The combined liquid mixture is then brought into contact with the solid phase.

After the sample has contacted the solid phase for a time sufficient so that first and second immuno-reactive substances have had a full opportunity to compete for combination with the antibody, then, in accordance with the present invention, the solid phase is isolated, bearing with it the second immuno-reactive substance still adsorbed to its surfaces and also bearing with it that proportion of the antibody which has been

-10-

bound by the second immuno-reactive substance. The isolation of the solid phase can be effected in any suitable manner. For example, in most instances the solid phase will comprise the walls and bottom of microtiter wells into which the liquid sample has been added, either simultaneously with the previously described reagent or after the reagent has been added to the sample. In any event, the sample and reagent will be present together in the titer well and isolation of the solid phase may merely consist of pouring the combined mixture of the liquid sample and reagent out of the well and then washing the well with distilled water or a selected buffer such as, for example, phosphate buffered saline (PBS) with a surfactant or the like, to substantially completely free it of the combined mixture of reagent and liquid sample.

Further, in accordance with the method of the present invention, the antibody which is bound to the passively adsorbed second immuno-reactive substance on the solid phase is labeled with any suitable selected labeling agent. For this purpose, one can utilize a radioactive labeling material such as $125_I$ or an enzyme-labeing material, for example, an enzyme labeled anti-globulin which in any event will attach to the antibody bound to the second immuno-reactive substance on the solid phase. Typical enzyme labeled anti-globulins comprise: alkaline phosphatase, peroxidase, glucose oxidase and $\beta$-D-galactose. The particular technique for effecting the labeling will depend upon the particular labeling material.

As one specific example, when a radioactive labeling reagent such as $^{125}I$ is used to label sheep anti-rabbit IgG, the labeled reagent having a specific activity of 300 to 1200 cpm/ng, can be added in a typical concentration of about 20 ng of active antibody per 250 µl PBS at room temperature to microtiter wells containing the solid phase hapten to which hapten specific rabbit antibody is bound. The contacting between the radioactive
labeled material and the antibody can be maintained, for
example, for about 30 minutes or until the serologic reaction
is complete. The radioactive reagent can then be removed, as
by decanting, and the microtiter well can be water-washed free
of traces of the same before the concentration of the radioactive
label remaining in the titer well is detected by conventional
radiation monitoring equipment, such as a geiger counter or the
like.

In the event that enzyme labeling is utilized, as a specific
example, alkaline phosphatase labeled sheep anti-rabbit IgG is
added in an aqueous solution in a concentration of about 250 ng
specific antibody/ml to a microtiter well containing solid phase
hapten to which rabbit hapten specific antibody is bound.
A specific volume, such as 250 μl of the reagent containing
the enzyme labeled anti-globulin reagent is used and a contact
time of, for example, 30 minutes at a temperature of 20-25°C is
employed. After the serologic reaction has been accomplished,
the concentration of the labeled agent bound to the antibody is
determined as a measure of the amount of antibody bound to the
solid phase and also as a measure of the first immuno-reactive
substance initially present in the sample. To accomplish this,
each microtiter well is filled with a specified volume of enzyme
substrate, for example, p-nitrophenyl phosphate or the like.
Hydrolysis of the substrate by enzymatic activity from the
enzyme covalently bound to the antibody is then measured
spectrophotometrically after the substrate has been incubated
in the microtiter well or a suitable period of time. For example,
the enzyme alkaline phosphatase attached covalently to sheep
anti-rabbit IgG in turn attached to the solid phase hapten
adsorbed to the surface of the walls of microtiter wells is

-12-

used to hydrolyze the substrate p-nitrophenyl phosphate.
The hydrolyzed substrate is then detected spectrophotometrically
as a measure of the antibody concentration. The results obtained
indicate the amount of hapten or antigen in the sample and also
the nature of the hapten or antigen. Further features of the
present invention are set forth in the following specific two
examples:

### EXAMPLE I

a.) Preparation of Digoxin - Human Serum Albumin Conjugate.

A human serum albumin-digoxin conjugate was prepared by
a periodate oxidation procedure described previously by
Smith et al (Biochemistry, 9: 331-337; 1970). Typically, 436
mg of digoxin was suspended in 20 ml of absolute ethanol at
room temperature to which was added dropwise with stirring
20 ml of 0.1M sodium metaperiodate. After 25 minutes, 0.6 ml
of 1.0M ethylene glycol was added. Following a five minute
incubation period, the reaction mixture was added dropwise
with stirring to 560 mg of human serum albumin dissolved in
20 ml of distilled $H_2O$ (pH previously adjusted to 9.5 with
5% $K_2CO_3$). This reaction mixture was maintained in the 9.0 -
9.5 pH range by the dropwise addition of $K_2CO_3$. After 45
minutes, 0.3g of sodium borohydride dissolved in 20 ml of
distilled $H_2O$ was added. Following a 3-hour incubation period,
1.0M formic acid was added dropwise to lower the pH to 6.5.
One hour later, the pH was raised to 8.5 by the addition of
1.0M $NH_4OH$. The reaction mixture was then dialyzed overnight
against cold running tap water. After 24 hours, the pH was
lowered to 4.5 by the dropwise addition of 0.1N HCl. The
reaction mixture was left at room temperature for one hour
and then placed at 4°C for four hours. The precipitated protein was collected by centrifugation at 1000X g. for one hour at 4°C. After discarding the supernatent, the precipitate was dissolved in a minimal amount of 0.19M $NaHCO_3$. This solution was then dialyzed against cold running tap water for four days and then lyophilized as human serum albumin-digoxin conjugate (HSA-D).

b.) Coating Polystyrene Microtiter Plates with HSA-D.

HSA-D was dissolved in 0.015M carbonate-bicvrbonate buffer (pH 9.5) at a concentration of 100 ng/ml. The wells of the plates were filled with 250 µl of the HSA-D solution, after which the plates were placed in a 4°C humid chamber and incubated overnight. On the following day, the liquid content of the wells was dumped out and the plates lyophilized for two hours. After lyophilization, the plates were packaged and sealed along with a dessicant in plastic bags for future use.

c.) Competitive Enzyme-Linked Immunosorbent Assay for Digoxin Using HSA-D Coatged Microtiter Plates.

In a typical assay, 50 µl of each serum sample was added to duplicate HSA-D coated wells to which was then added µl of an appropriate dilution of rabbit anti-digoxin prepared in phosphate buffered saline with Tween (PBS-Tween). After a 30-minute incubation period, the wells were washed three times with PBS-Tween and then filled with 250 µl of an appropriate dilution of alkaline phosphate conjugated sheep anti-rabbit IgG. Following a 30-minute incubation period at room temperature, unreacted conjugate was removed by washing the wells with PBS-Tween three times. The serologic reaction was then developed by adding 250 µl of enzyme substrate (1.0 mg p-nitrophenyl phosphate/ml of pH 9.8 diethanolamine buffer) to each well and incubating at room temperature for 45 minutes. The enzymatic reaction was

-14-

stopped at this point by the addition of 50 μl of 3 N NaOH to each well. The reaction was meaured spectrophotometrically by determining the absorbance value for the contents in each well at 405 nm. The mean value for each of the digoxin standards was computed and used to construct the standard curve set forth below (Fig. 1). The amount of digoxin in the unknown samples was determined from that standard curve.

Fig. 1 - Inhibition curve for digoxin

EXAMPLE II

a.) Preparation of Thyroxin-Bovine Serum Albumin Conjugate.

A bovine serum albumin-thyroxin conjugate was prepared by a procedure which employed glutaraldehyde coupling reagent. The conjugation was initiated by mixing equal volumes of a 0.009 mM solution of bovine serum albumin (BSA) containing 9.02M PBS, pH 7.6 and a 0.125 mM solution of thyroxin (.01M PBS, pH adjusted to 10 with 1N NaOH). After mixing at room temperature for two hours, a 25% solution of gluteraldehyde was added to a final concentration of 0.2%. This reaction mixture was stirred gently at room temperature for two hours and then dialyzed for three days against a minimum of 100 volumes of 0.01M PBS (pH 7.6) changed at

-15-

24-hour intervals. The conjugation protocol was completed by a final 24-hour dialysis against 0.05M Tris buffer at pH 8.0. The final product was stored at -20°C for future use.

b.) Coating Polystyrene Cuvettes.

BSA-thyroxin was dissolved in 0.015M carbonate bicarbonate buffer (pH 9.5) at a concentration of 200 ng/ml. The wells of the plates were filled with 250 µl of the BSA-thyroxin solution, after which the plates were placed in a 4°C humid chamber and incubated overnight. On the following day, the liquid contents of the wells were dumped out and the plates lyophilized for two hours. After lyophilization, the plates were packaged and sealed along with a dessicant in plastic bags for future use.

c.) Competitive Enzyme-Linked Immunosorbent Assay for Thyroxin Using BSA-Thyroxin Coated Microtiter Plates.

In a typical assay, 10 µl of each unknown serum sample was added to duplicate BSA-Thyroxin coated wells to which 250 µl of an appropriate dilution of rabbit anti-thyroxine prepared in PBS-Tween. After a 30-minute incubation period, the wells were washed three times with PBS-Tween and then filled with 250 µl of an appropriate dilution of alkaline phosphatase conjugated sheep anti-rabbit IgG. Following a 30-minute incubation period at room temperature, unreacted conjugate was removed by washing the wells with PBS-Tween three times. The serologic reaction was then developed by adding 250 µl of enzyme substrate (1.0 mg p-nitrophenyl phosphate/ml of pH 9.8 diethanolamine buffer) to each well and incubating at room temperature for 45 minutes. The enzymatic reaction was stopped at this point by the addition of 50 µl of 3N NaOH to each well. The reaction was measured spectrophotometrically be determining the
absorbance value for the contents in each well at 405 nm.
The mean value for each of the thyroxin standards was
computed and used to construct a standard curve which
appears below ( Fig. 2). The amount of thyroxin in the
unknown samples was determined from that standard curve.

Fig. 2 - Inhibition curve for thyroxine

Examples I and II set forth in paragraphs above clearly illustrate that the method of the present invention is simple, inexpensive and effective for determining the presence and the concentration of selected antigens and haptens in a safe, rapid and sensitive manner. The method employs solid phases to which selected immuno-reactive haptens or antigens have been adsorbed. The method is equally adaptable to labeling by the enzyme or radioactive procedure or, for that matter, coloro-metrically or in other ways. The increased sensitivity of the method in comparison with those which employ labeled haptens is at least about 2 to 4 times. The technical difficulties of covalently binding haptens to enzymes while retaining enzymatic activity is obviated. Moreoever, the method can easily be carried out in standard microtiter plates and other conventional analytical equipment. Various other advantages are as set forth in the foregoing.

Various modifications, changes, alterations and additions can be made in the present method and in its steps and their parameters.

Such modifications, changes, alterations and additions as are within the scope of the appended claims form part of the present invention.

What is claimed is:

1. An improved method of detecting and quantitating haptens and antigens, said method comprising:

    a. contacting a sample containing an unknown quantity of a first immuno-reactive substance selected from the group consisting of haptens, antigens and mixtures thereof, to be detected and quantitated, with

        i. a known quantity of a reagent containing a known concentration of an antibody specific for said first immuno-reactive substance, and

        ii. a solid phase having a known concentration of a second immuno-reactive substance selected from the group consisting of haptens, antigens and mixtures thereof reactible with said antibody passively adsorbed thereto, said contacting being effected for a time sufficient to cause said first and second immuno-reactive substances to combine competitively with said antibody;

    b. isolating said solid phase while still retaining said second immuno-reactive substance passively adsorbed thereto;

    c. labeling the antibody bound to said passively adsorbed second immuno-reactive substance on said solid phase with a selected labeling agent; and,

    d. detecting the concentration of said selected labeling agent bound to said antibody as a measure of the amount of antibody bound to said solid phase and also as a measure of said first immuno-reactive substance initially present in said sample.

-1-

0028133

2. The improved method of claim 1 wherein said sample is in the liquid phase and is utilized in a specific volume and wherein said reagent containing said antibody is in the liquid phase, and wherein said liquid phase is separated from said solid phase to isolate said solid phase.

3. The improved method of claim 2 wherein said liquid sample and said reagent containing said antibody are mixed together and react before the resulting liquid phase mixture contacts said solid phase.

4. The improved method of claim 2 wherein said liquid sample, said liquid reagent containing said antibody and said solid phase initially contact each other substantially simultaneously.

5. The improved method of claim 2 wherein said labeling is effected through the use of an enzyme.

6. The improved method of claim 5 wherein said detection of said concentration of said enzyme is effected by hydrolyzing said enzyme from attachment to said antibody on said solid phase and determining the concentration of said hydrolyzed enzyme.